(19) Europäisches Patentamt
European Patent Office
Office européen des brevets

(11) Veröffentlichungsnummer: **0 024 570**
**B1**

(12) # EUROPÄISCHE PATENTSCHRIFT

(45) Veröffentlichungstag der Patentschrift: **12.05.82**

(51) Int. Cl.³: **C 07 D 233/88**, A 01 N 43/50

(21) Anmeldenummer: **80104520.4**

(22) Anmeldetag: **31.07.80**

(54) **5-Imino-imidazolin-2-one, diese enthaltende Herbizide und Verfahren zur Bekämpfung unerwünschter Pflanzen mit diesen Verbindungen.**

(30) Priorität: **22.08.79 DE 2933917**

(43) Veröffentlichungstag der Anmeldung: **11.03.81 Patentblatt 81/10**

(45) Bekanntmachung des Hinweises auf die Patenterteilung: **12.05.82 Patentblatt 82/19**

(84) Benannte Vertragsstaaten: **AT BE CH DE FR GB IT LI NL SE**

(56) Entgegenhaltungen:
**EP-A-0 001 813**
**DE-A-2 251 354**
**FR-A-2 002 196**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Oeser, Heinz-Guenter, Dr., Friedrich-Burschell-Weg 19, D-6700 Ludwigshafen (DE)**
Erfinder: **Jacobs, Peter, Dr., Hanns-Fay-Strasse 4, D-6701 Frankenthal (DE)**
Erfinder: **Wuerzer, Bruno, Dr., Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 024 570 B1**

BUNDESDRUCKEREI BERLIN

# 5-Imino-imidazolidin-2-one, diese enthaltende Herbizide und Verfahren zur Bekämpfung unerwünschter Pflanzen mit diesen Verbindungen

Die vorliegende Erfindung betrifft wertvolle neue 5-Imino-imidazolidin-2-one, Herbizide, welche diese Verbindungen als Wirkstoffe enthalten sowie Verfahren zur Bekämpfung unerwünschten Pflanzenwuchses mit diesen Verbindungen.

Es ist bekannt, 4-Alkyl-5-imino-imidazolidin-2-one (BE-PS 728 537) als Herbizide zu verwenden. Die Beispiele der Patentschrift betreffen die Bekämpfung weniger breitblättriger unerwünschter Pflanzen bei verschiedenen Getreidearten, Karotten und Lein.

Es wurde nun gefunden, daß 5-Imino-imidazolidin-2-one der allgemeinen Formel

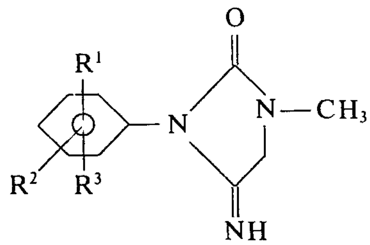

in welcher $R^1$, $R^2$ und $R^3$ gleich oder verschieden sind und unabhängig voneinander Wasserstoff, Halogen (z. B. Fluor, Chlor, Brom oder Jod), eine gegebenenfalls halogensubstituierte Alkyl-, Alkoxy- oder Alkyl-thio-Gruppe mit jeweils 1 bis 4 C-Atomen (z. B. Methyl, Ethyl, 2-Chlorethyl, 1-Chlor-2-propyl, Isopropyl, tert.-Butyl, Methoxy, Ethoxy, tert.-Butoxy, Trifluormethyl, Tetrafluorethoxy, Methyl-thio), Can oder Nitro bedeuten, vorzugsweise bei Vorlaufanwendung eine Reihe landwirtschaftlich wichtiger breitblättriger Unkräuter und unerwünschter Gräser bekämpfen und gleichzeitig wichtige Kulturpflanzen, beispielsweise Erdnüsse, Soja oder Kultursorghum, nicht schädigen.

Als Beispiele für die neuen Wirkstoffe seien im folgenden einzelne Verbindungen genannt:

1-Phenyl-3-methyl-5-imino-imidazolidin-2-on,
1-(2-Fluorphenyl)-3-methyl-5-imino-imidazolodin-2-on,
1-(2-Chlorphenyl)-3-methyl-5-imino-imidazolidin-2-on,
1-(3-Chlorphenyl)-3-methyl-5-imino-imidazolidin-2-on,
1-(4-Chlorphenyl)-3-methyl-5-imino-imidazolidin-2-on,
1-(4-Ethyl-phenyl)-3-methyl-5-imino-imidazolidin-2-on,
1-[(4-(2-Chlorethyl)-phenyl]-3-methyl-5-imino-imidazolodin-2-on,
1-[3-(1,1,2,2-Tetrafluor-ethoxy)-phenyl]-3-methyl-5-imino-imidazolidin-2-on,
1-(3-Isopropyl-phenyl)-3-methyl-5-imino-imidazolidin-2-on,
1-(4-Isopropyl-phenyl)-3-methyl-5-imino-imidazolidin-2-on,
1-[4-(1-Chlor-2-isopropyl)-phenyl]-3-methyl-5-imino-imidazolidin-2-on,
1-(3-tert.-Butyl-phenyl)-3-methyl-5-imino-imidazolidin-2-on,
1-(4-tert.-Butyl-phenyl)-3-methyl-5-imino-imidazolidin-2-on,
1-(3,4-Difluor-phenyl)-3-methyl-5-imino-imidazolidin-2-on,
1-(3-Chlor-4-fluor-phenyl)-3-methyl-5-imino-imidazolidin-2-on,
1-(3,4-Dichlor-phenyl)-3-methyl-5-imino-imidazolidin-2-on,
1-(3,5-Dichlor-phenyl)-3-methyl-5-imino-imidazolidin-2-on,
1-(3-Brom-4-Chlor-phenyl)-3-methyl-5-imino-imidazolidin-2-on,
1-(3-Chlor-4-methyl-phenyl)-3-methyl-5-imino-imidazolidin-2-on,
1-(3-Chlor-4-methoxy-phenyl)-3-methyl-5-imino-imidazolidin-2-on,
1-(3-Chlor-4-methyl-thio-phenyl)-3-methyl-5-imino-imidazolidin-2-on,
1-(3-Trifluormethyl-4-chlor-phenyl)-3-methyl-5-imino-imidazolidin-2-on,
1-(3-Chlor-4-ethoxy-phenyl)-3-methyl-5-imino-imidazolidin-2-on,
1-(3-Nitro-4-methyl-phenyl)-3-methyl-5-imino-imidazolidin-2-on,
1-(3,5-Dichlor-4-methoxy-phenyl)-3-methyl-5-imino-imidazolidin-2-on,
1-(3-Chlor-4-methoxy-5-brom-phenyl)-3-methyl-5-imino-imidazolidin-2-on.

Die neuen Wirkstoffe der Formel I können erhalten werden, wenn man beispielsweise aromatische Isocyanate der allgemeinen Formel

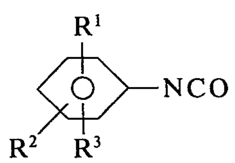

II

in welcher $R^1$, $R^2$ und $R^3$ die oben angegebene Bedeutung haben, mit Methylaminoacetonitril ($CH_3NHCH_2CN$) gegebenenfalls in Gegenwart von aromatischen Kohlenwasserstoffen, von chlorierten aliphatischen und aromatischen Kohlenwasserstoffen oder von Ethern, beispielsweise Diethyl- oder Dibutylether, Dioxan und Tetrahydrofuran oder von Ketonen, z. B. Aceton und Methylethylketon als Verdünnungsmittel und gegebenenfalls in Gegenwart eines basischen Katalysators, z. B. Triethylamin, N-Methylpiperidin und N,N-Dimethylcyclohexylamin umsetzt. Ferner erhält man dieselbe Verbindung der Formel I, wenn man beispielsweise ein aromatisches Isocyanat der Formel II gegebenenfalls gelöst in einem mit Wasser nicht mischbaren organischen Lösungsmittel mit der wäßrigen Lösung von Methylamino-acetonitril-Hydrochlorid gegebenenfalls in Gegenwart eines 1,1- bis 2molaren Überschusses (bezogen auf das Hydrochlorid) an Natriumhydroxid, Natriumcarbonat, Kaliumhydroxid, Kaliumcarbonat, Triethylamin oder Pyridin umsetzt.

Die oben beschriebenen Umsetzungen werden bei Temperaturen von etwa −10 bis +80°C, vorzugsweise 0 bis +40°C, durchgeführt.

Die wahrscheinlich als Zwischenstufen auftretenden 1-Aryl-3-methyl-3-cyanmethylharnstoffe der Formel

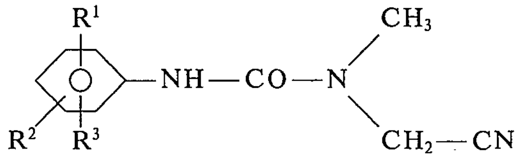

III

in welcher $R^1$, $R^2$ und $R^3$ die oben angegebene Bedeutung haben, cyclisieren spontan zu den neuen Verbindungen der Formel I.

Die als Ausgangsstoffe verwendeten Isocyanate der Formel II sind bekannt oder lassen sich nach üblichen Verfahren leicht herstellen.

Das als Ausgangsverbindung verwendete Methylamino-acetonitril kann nach dem in der deutschen Offenlegungsschrift 2 503 582 beschriebenen Verfahren hergestellt werden.

Die neuen Verbindungen der Formel I sind farblose kristalline Produkte, leicht löslich in Essigester, Aceton, Ethanol, Tetrahydrofuran, Methylenchlorid, Chloroform, Dimethylsulfoxid, Dimethylformamid und N-Methylpyrrolidon.

Die folgenden Versuchsangaben erläutern die Herstellung der Wirkstoffe.

Beispiel 1

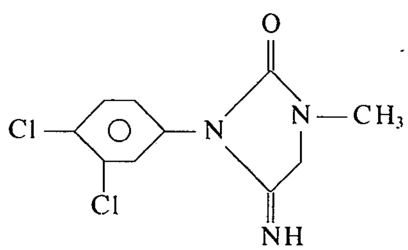

Zu einer Lösung von 3,5 g (0,05 Mol) Methylamino-acetonitril in 50 ml trockenem Toluol werden 0,2 g Triethylamin zugesetzt und anschließend bei 10 bis 15°C eine Lösung von 9,4 g (0,05 Mol) 3,4-Dichlorphenylisocyanat in 50 ml trockenem Toluol zugetropft. Das Gemisch wird über Nacht gerührt und der Niederschlag abgesaugt, mit je 50 ml Toluol und Petrolether gewaschen und getrocknet. Man erhält 12 g (93% d. Th.) 1-(3,4-Dichlorphenyl)-3-methyl-5-imino-imidazolidin-2-on als weiße, analysenreine Kristalle vom Schmelzpunkt 158 bis 161°C (Wirkstoff Nr. 1).

Beispiel 2

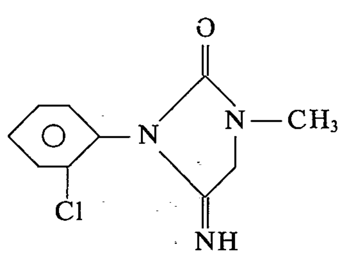

Eine Lösung von 18,6 g (0,175 Mol) Methylamino-acetonitril-hydrochlorid in 70 ml Wasser wird bei 10°C mit 50 ml Methylenchlorid und mit der Lösung von 7 g Natriumhydroxid in 20 ml Wasser versetzt.

Dem gut gerührten Gemisch wird bei 10 bis 15°C die Lösung von 20,5 g (0,15 Mol) o-Chlorphenylisocyanat in 100 ml trockenem Methylenchlorid zugetropft. Nach dreistündigem Nachrühren bei Raumtemperatur wird die organische Phase getrennt, zweimal mit je 75 ml Wasser gewaschen und eingeengt. Der Rückstand wird mit 75 ml Petrolether gelöst, abgesaugt und mit Petrolether gewaschen. Man erhält 31,2 g (92% d. Th., bezogen auf das eingesetzte Isocyanat) 1-(2-Chlorphenyl)-3-methyl-5-imino-imidazolidin-2-on als weiße Kristalle vom Schmelzpunkt 138 bis 141°C (Wirkstoff Nr. 2).

Analog werden die in er folgenden Tabelle 1 aufgeführten Verbindungen hergestellt.

Tabelle 1

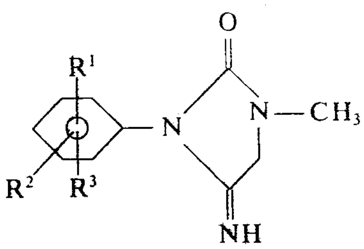

| Wirkstoff Nr. | $R^1$ | $R^2$ | $R^3$ | Schmelzpunkt °C |
|---|---|---|---|---|
| 3 | H | H | H | 113–116 |
| 4 | 2-F | H | H | 135–138 |
| 5 | 3-Cl | H | H | 107–110 |
| 6 | H | 4-Cl | H | 108–110 |
| 7 | H | 4-$C_2H_5$— | H | 78– 80 |
| 8 | H | 4-(Cl—$CH_2CH_2$—) | H | Harz |
| 9 | 3-($CF_2H$—$CF_2$—O—) | H | H | 63– 64 |
| 10 | 3-(i-$C_3H_7$) | H | H | 90– 92 |
| 11 | H | 4-(i-$C_3H_7$) | H | 110–112 |
| 12 | H | 4-(Cl—$CH_2$—CH—$CH_3$) | H | 104–106 |
| 13 | 3-(tert.-$C_4H_9$—) | H | H | 82– 84 |
| 14 | H | 4-(tert.-$C_4H_9$) | H | 111–113 |
| 15 | 3-F | 4-F | H | 102–104 |
| 16 | 3-Cl | 4-F | H | 146–150 |
| 17 | 3-Cl | H | 5-Cl | 118–120 |
| 18 | 3-Br | 4-Cl | H | 94– 96 |
| 19 | 3-Cl | 4-$CH_3$— | H | 102–104 |
| 20 | 3-Cl | 4-$CH_3$—O— | H | 142–145 |
| 21 | 3-Cl | 4-$CH_3$—S— | H | 142 (Zers.) |
| 22 | 3-$CF_3$ | 4-Cl | H | 76– 78 |
| 23 | 3-Cl | 4-$C_2H_5$—O— | H | 104–106 |
| 24 | 3-$NO_2$ | 4-$CH_3$— | H | 174–175 |
| 25 | 3-Cl | 4-$CH_3O$— | 5-Cl | 121–123 |
| 26 | 3-Cl | 4-$CH_3O$— | 5-Br | 92– 95 |

Die Anwendung der Wirkstoffe erfolgt z. B. in Form von direkt versprühbaren Lösungen, Pulvern, Suspensionen — auch hochprozentige wäßrige, ölige oder sonstige Suspensionen — oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw. sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw. stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz- Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäuren, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctyl-phenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxylierten Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit ienem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerde wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% Wirkstoff, vorzugsweise zwischen 0,5 und 90 Gew.-%.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Der Einfluß verschiedener Vertreter der neuen Verbindungen auf das Wachstum von unerwünschten Pflanzen im Vergleich zu bekannten Wirkstoffen wird in nachfolgenden Gewächshausversuchen demonstriert.

Als Kluturgefäße dienten Plastikblumentöpfe mit 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorauflaufwendungen das Aufbringen der Wirkstoffe auf die Erdoberfläche. Sie wurden hierbei in Wasser als Verteilungsmittel suspendiert oder emulgiert und mittels fein verteilender Düse gespritzt. Nach dem Aufbringen der Mittel wurden die Gefäße leicht beregnet, um Keimung und Wachstum in Gang zu bringen und auch gleichzeitig die Wirkstoffe zu aktivieren. Danach deckte man die Gefäße mit durchsichtigen Plastikhauben ab, bis die Pflanzen angewachsen waren. Die Abdeckung bewirkte ein gleichmäßiges Keimen der Testpflanzen, sofern dies nicht durch die Wirkstoffe beeinträchtigt wurde.

Zum Zecke der Nachauflaufbehandlung zog man die Pflanzen je nach Wuchsform in den Versuchsgefäßen erst bis zu einer Höhe von 3 bis 10 cm an und behandelte sie danach. Eine Abdeckung unterblieb. Die Aufstellung der Gefäße erfolgte im Gewächshaus, wobei für wärmeisolierende Arten heißere Bereiche (25 bis 40°C) und für solche gemäßigter Klimate 15 bis 30°C bevorzgut wurden. Die Versuchsperiode erstreckte sich über 3 bis 6 Wochen. Während dieser Zeit

wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha Aktivsubstanz und die Testpflanzenarten.

Bewertet wird nach einer Skala von 0 bis 100.

Dabei bedeutet 0 keine Schädigung oder normalen Ablauf und 100 kein Aufgang der Pflanzen bzw. völlige Zerstörung zumindest der oberirdischen Sproßteile.

Die beigefügten Tabellen zeigen die selektive herbizide Wirkung der neuen Verbindungen. Dabei eröffnen einige der neuen Verbindungen Anwendungsmöglichkeiten in Kulturen, welche den bekannten Wirkstoffen dieser Stoffklasse wegen unzulänglicher Verträglichkeit verschlossen blieben. Die Applikation erfolgt im Nachlaufverfahren, vorzugsweise aber im Vorlaufverfahren, während die bekannten Verbindungen ihre Stärke bei Nachauflaufanwendung haben. Die Mittel können auf den Standort aufgebracht werden bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Eine weitere Ausbringungstechnik besteht darin, daß die Wirkstoffe mit Hilfe der Spritzgeräte so gespritzt werden, daß die Blätter empfindlicher Kulturpflanzen nach Möglichkeit nicht getroffen werden, wähernd die Wirkstoffe auf die darunterliegende Bodenfläche oder dort wachsende unerwünschte Pflanzen gelangen (post-directed, lay-by). Die Aufwandmengen betragen je nach Jahreszeit und Wachstumsstadium 0,1 bis 15 kg/ha Wirkstoffmenge und mehr, wobei sich die höheren Aufwandmengen bevorzugt zur totalen Bekämpfung von Vegetationen eignen.

In Anbetracht der Vielseitigkeit der Applikationsmethoden können die erfindungsgemäßen Mittel oder diese enthaltende Mischungen außer bei den in den Tabellen aufgeführten Nutzpflanzen noch in einer weiteren großen Zahl von Kulturen zur Beseitigung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativs | Hafer | cats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellis sinensis | Teestrauch | tea plants |
| Carthamus tinctorius | Saflor — Färbedistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |

Fortsetzung

| Botanischer Name | Deutscher Name | Englischer Name |
|---|---|---|
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs an lawns |
| Daucus carota | Möhre | carrots |
| Elaeis guineensis | Ölpalme | oil palms |
| Fragaria vasca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoes batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esulenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Oles europaea | Ölbaum | olive trees |
| Oryza sativa | Reis | rice |

Fortsetzung

| Botanischer Name | Deutscher Name | Englischer Name |
| --- | --- | --- |
| Panicum miliaceum | Rispenhirse | |
| Phaseolus lunatus | Mondblume | limabeans |
| Phaseolus mungo | Urdbohne | mungsbeans |
| Phaseolus vulgaris | Buschbohne | snapbeans, green beans, dry beans |
| Pennisetum glaucum | Perl- oder Rohrkolbenhirse | |
| Tetroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pera trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |

Fortsetzung

| Botanischer Name | Deutscher Name | Englischer Name |
|---|---|---|
| Vigna sinensis (V. unguiculata) | Kuhbohne | cow peas |
| Vitis vinifera | Weinrebe | grapes |
| Zea mays | Mais | Indian corn, sweet corn, maize |

Die neuen 5-Imino-imidazolidin-2-one können unter sich und mit zahlreichen Vertretern anderer herbizider oder wachstumsregulierender Wirkstoffgruppen gemischt und gemeinsam ausgebracht werden. Beispielsweise kommen als Mischungskomponenten Diazine, N-Phenyl-carbamate, Thiolcarbamate, Diurethane, Halogencarbonsäure, Phenoxyfettsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte. Eine Reihe von Wirkstoffen, welche zusammen mit den neuen Verbindungen für verschiedenste Anwendungsbereiche sinnvolle Mischungen ergeben, werden beispielhaft aufgeführt. Dabei bringen Nachauflaufherbizide eine besondere Bereicherung, indem diese die Wirkung über das Blatt bringen, während sich die erfindungsgemäßen Verbindungen durch ihre Wirkung über den Boden auszeichnen.

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-m.trifluormethylphenyl-3(2H)-pyridazinon
5-Methylamino-4-chlor-2-m-α,α,β,β-tetrafluorethoxyphenyl-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-m.trifluormethylphenyl-3(2H)-pyridazinon
5-Methoxy-4-chlor-2-m.trifluormethylphenyl-3(2H)-pyridazinon
5-Amino-4-brom-2-m.methylphenyl-3(2H)-pyridazinon
3-(1-Methyläthyl)-1H-2,1,3-benzotiadiazin-4(3H)-on-2,2-dioxid und Salze
3-(1-Methyläthyl)-8-chlor-1H-2,1,3-benzothiadiazin-4(3H)-on-2,2-dioxid und Salze
3-(1-Methyläthyl)-8-fluor-1H-2,1,3-benzothiadiazin-4(3H)-on-2,2-dioxid und Salze
3-(1-Methyläthyl)-8-methyl-1H-2,1,3-benzothiadiazin-4(3H)-on-2,2-dioxid und Salze

1-Methoxymethyl-3-(1-methyläthyl)-2,1,3-benzothiadiazin-4(3H)-on-2,2-dioxid
1-Methoxymethyl-8-chlor-3-(1-methyläthyl)-2,1,3-benzothiadiazin-4(3H)-on-2,2-dioxid
1-Methoxymethyl-8-fluor-3-(1-methyläthyl)-2,1,3-benzothiadiazin-4(3H)-on-2,2-dioxid
1-Cyan-8-chlor-3-(1-methyläthyl)-2,1,3-benzothiadiazin-4(3H)-on-2,2-dioxid
1-Cyan-8-fluor-3-(1-methyläthyl)-2,1,3-benzothiadiazin-4(3H)-on-2,2-dioxid
1-Cyan-8-methyl-3-(1-methyläthyl)-2,1,3-benzothiadiazin-4(3H)-on-2,2-dioxid
1-Cyan-3-(1-methyläthyl)-2,1,3-benzothiadiazin-4(3H)-on-2,2-dioxid
1-Azidomethyl-3-(1-methyläthyl)-2,1,3-benzothiadiazin-4(3H)-on-2,2-dioxid
3-(1-methyläthyl)-1H-(pyridino-[3,2-e]2,1,3-thiadiazin-(4)-on-2,2-dioxid

N-(1-Äthylpropyl)-2,6-dinitro-3,4-dimethylanilin
N-(1-Methyläthyl)-N-äthyl-2,6-dinitro-4-trifluormethylanilin
N-n.Propyl-N-β-chloräthyl-2,6-dinitro-4-trifluormethyl-anilin
N-n.Propyl-N-cyclopropylmethyl-2,6-dinitro-4-trifluormethyl-anilin
N-Bis(n.propyl)-2,6-dinitro-3-amino-4-trifluormethylanilin
N-Bis(n.propyl)-2,6-dinitro-4-methyl-anilin
N-Bis(n.propyl)-2,6-dinitro-4-methylsulfonyl-anilin
N-bis(n.propyl)-2,6-dinitro-4-aminosulfonyl-anilin
Bis(β-chloräthyl)-2,6-dinitro-4-methyl-anilin
N-Äthyl-N-(2-methylallyl)-2,6-dinitro-4-trifluormethyl-anilin

N-Methylcarbaminsäure-3,4-dichlorbenzylester
N-Methylcarbaminsäure-2,6-di(tert.butyl)-4-methylphenylester
N-Phenylcarbaminsäure-isopropylester
N-3-Fluorphenylcarbaminsäure-3-methoxypropyl-2-ester

N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Äthyl-2-(phenylcarbamoyl)-oxypropionsäureamid
3'-N-Isopropyl-carbamoyloxy-propionanilid

Äthyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-äthyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Äthyl-N-(3-N'-3-chlor-4-fluorphenylcarbamoyloxy)-phenyl)-carbamat
Äthyl-N-(3-N'-3,4difluorphenylcarbamoyloxy)-phenyl)-carbamat
Methyl-N-(3-(N'-3,4difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-äthylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester

N,N-Diäthyl-thiolcarbaminsäure-p-chlorbenzylester
N,N-Di-n.propyl-thiolcarbaminsäure-äthylester
N,N-Di-n.propyl-thiolcarbaminsäure-n.propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-äthyl-5-isoxazolyl-methylester
N,N-Di-sec.butyl-thiolcarbaminsäure-äthylester
N,N-Di-sec.butyl-thiolcarbaminsäure-benzylester
N-Äthyl-N-cyclohexyl-thiolcarbaminsäure-äthylester
N-Äthyl-N-bicyclo-[2,2,1]-heptyl-thiolcarbaminsäure-äthylester
S-(2,3-Dichlorallyl)-2,2,4-trimethyl-azetidin)-1-carbothiolat
S-(2,3,3-Trichlorallyl)-2,2,4-trimethyl-azetidin)-1-carbothiolat
S-Äthyl-hexahydro-1-H-azepin-1-carbothiolat
S-Benzyl-3methylhexahydro-1-H-azepin-1-carbothiolat
S-Benzyl-2,3-dimethylhexahydro-1-H-azepin-1-carbothiolat
S-Äthyl-3methylhexahydro-1-H-azepin-1-carbothiolat

N-Äthyl-N-n.butyl-thiolcarbaminsäure-n.propylester
N,N-Dimethyl-dithiocarbaminsäure-2-chlorallylester
N-Methyl-dithiocarbaminsäure-Natrium
Trichloressigsäure-Na salz
α,α-Dichlorpropionsäure-Na salz
α,α-Dichlorbuttersäure-Na salz
α,α,β,β-Tetrafluorpropionsäure-Na salz
α-Methyl,α,β-dichlorpropionsäure-Na salz
α-Chlor-β-(4-chlorphenyl)-propionsäure-methylester
α,β-Dichlor-β-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureäthylester

2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Na salz
2-[4-(3',5'-Dichlorpyridyl-2-oxy)-phenoxy]-propionsäure-Na salz

2-(N-Benzoyl-3,4-dichlorphenylamino)-propionsäureäthylester
2-(N-Benzoyl-3-chlor-4-fluorphenylamino)-propionsäure-methylester
2-(N-Benzoyl-3-chlor-4-fluorphenylamino)-propionsäure-isopropylester

2-Chlor-4-äthylamino-6-isopropylamino-1,3,5-triazin
2-Chlor-4-äthylamino-6-(amino-2'-propionitril)-1,3,5-triazin
2-Chlor-4-äthylamino-6-2-methoxypropyl-2-amino-1,3,5-triazin
2-Chlor-4-äthylamino-6-butin-1-yl-2-amino-1,3,5-triazin
2-Chlor-4,6-bisäthylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-äthylamino-6-isopropylamino-1,3,5-triazin
2-Methylthio-4-äthylamino-6-tert.butylamino-1,3,5-triazin
2-Methylthio-4,6-bisäthylamino-1,3,5-triazin
2-Methylthio-4,6-bisisopropylamino-1,3,5-triazin

2-Methoxy-4-äthylamino-6-isopropylamino-1,3,5-triazin
2-Methoxy-4,6-bisäthylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-äthan (Salze)
[-1-(1,2,4-Triazolyl-1')]-[1(4'-chlorphenoxy)]-3,3-dimethylbutan-2-on
N,N-Diallylchloracetamid
N-Isopropyl-2-chloracetanilid
N-(Butin-1-yl-3)-2-chloracetanilid

2-Methyl-6-äthyl-N-(propargyl)-2-chloracetanilid
2-Methyl-6-äthyl-N-(äthoxymethyl)-2-chloracetanilid
2-Methyl-6-äthyl-N-(2-methoxy-1-methyläthyl)-2-chloracetanilid
2-Methyl-6-äthyl-N-(isopropoxycarbonyläthyl)-2-chloracetanilid
2-Methyl-6-äthyl-N-(4-methoxypyrazol-1-yl-methyl)-2-chloracetanilid
2-Methyl-6-äthyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxalan-2-yl-methyl)-2-chloracetanilid
2,6-Dimethyl-N-(2-methoxyäthyl)-2-chloracetanilid
2,6-Dimethyl-N-(isobutoxymethyl)-2-chloracetanilid
2,6-Diäthyl-N-(methoxymethyl)-2-chloracetanilid
2,6-Diäthyl-N-(n.butoxymethyl)-2-chloracetanilid
2,6-Diäthyl-N-(äthoxycarbonylmethyl)-2-chloracetanilid

2,3,6-Trimethyl-N-(pyrazol-1-yl-methyl)-2-chloracetanilid
2,3-Dimethyl-N-(isopropyl)-2-chloracetanilid
2,6-Diäthyl-N-(propoxyäthyl)-2-chloracetanilid

2-(2-Methyl-4-chlorphenoxy-N-methoxy-acetamid
2-(α-Naphtoxy)-N,N-diäthylpropionamid
2,2-Diphenyl-N,N-dimethylacetamid
α(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-äthoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4,-dinitrophenylbenzaldoxim (Salze)
3,5-Dibrom-4-hydroxy-O-2-Cyan-4-nitrophenylbenzaldoxim (Salze)
Pentachlorphenol-Natriumsalz
2,4-Dichlorphenyl-4'-nitrophenyläther
2,4,6-Trichlorphenyl-4'-nitrophenyläther
2-Fluor-4,6-dichlorphenyl-4'-nitrophenyläther
2-Chlor-4-trifluormethylphenyl-4'-nitrophenyläther
2,4'-Dinitro-4-trifluormethyl-diphenyläther
2,4-Dichlorphenyl-3'-methoxy-4'-nitro-phenyläther
2-Chlor-4-trifluormethylphenyl-3'-äthoxy-4'-nitro-phenyläther
2-Chlor-4-trifluormethylphenyl-3'-carboxy-4'-nitro-phenyläther (Salze)
2,4-Dichlorphenyl-3'-methoxycarbonyl-4'-nitro-phenyläther
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso.Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2.6}$,$0^{8.11}$]-dodeca-3,9-dien
2-Äthoxy-2,3-dihydro-3,3-dimethyl-5-benzofuranyl-methan-sulfonat
2-Äthoxy-2,3-dihydro-3,3-dimethyl-5-benzofuranyl-dimethyl-aminosulfonat
2-Äthoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureamid
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-chlorphenyl)-1-benzoyl-3,3-dimethyl-harnstoff
1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n.butyl-harnstoff

1-(4-i-Propylphenyl)-3,3-dimethyl-harnstoff
1-(3-Trifluormethylphenyl)-3,3-dimethyl-harnstoff
1-(3-α,α,β,β-Tetrafluoräthoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooktyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Äthylen-2,2'-dipyridylium-dibromid
3-[1(N-Äthoxyamino)-propyliden]-6-äthyl-3,4-dihydro-2-H-pyran-2,4-dion
3-[1-(N-Allyloxyamino)-propyliden]-6-äthyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibberellinsäure (Salze)
Dinatrium-methylarsonat
Mononatriumsalz der Methylarsonsäure

N-Phosphon-methyl-glycin (Salze)
N,N-Bis(phosphonmethyl)-glycin (Salze)
2-Chloräthanphosphonsäure-2-chloräthylester
Ammonium-äthyl-carbamoyl-phosphonat
Di-n.butyl-1-n.butylamino-cyclohexyl-phosphonat
Trithiobutylphosphit
O,O-Diisopropyl-5-(2-benzosulfonylamino-äthyl)-phosphordithionat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chloräthyl)-trimethyl-ammoniumchlorid
(2-Methyl-4-phenylsulfonyl)-trifluormethansulfonanilid
1,1-Dimethyl-4,6-diisopropyl-5-indanyläthylketon
Natriumchlorat
Ammoniumrhodanid
Calciumcyanamid
2-Chlor-4-trifluormethylphenyl-3'-äthoxycarbonyl-4'-nitrophenyläther
1-(4-Benzyloxyphenyl)-3-methyl-3-methoxyharnstoff
2-[1-(2,5-Dimethylphenyl)-äthylsulfonyl]-pyridin-N-oxid

Außerdem ist es nützlich, die neuen Verbindungen allein oder in Kombinationen mit anderen Herbiziden auch noch mit weiteren Pflanzenschutzmitteln gemischt gemeinsam anzubringen, beispielsweise mit Mitteln zur Bekämpfung von Schädlingen oder phytopathogenen Pilzen bzw. Bakterien. Von Interesse sind ferner die Mischungen mit Mineralstofflösungen, welche zur Behebung von Ernährungs- oder Spurenelementmängeln eingesetzt werden. Zu den Einzelwirkstoffen oder Mischungen können auch die Öle verschiedenen Typs, Ölkonzentrate, Netz- oder Haftmittel sowie Antischaummittel zugesetzt werden.

Tabelle 1

Liste der Pflanzennamen

| Botanischer Name | Deutscher Name | Englischer Name |
|---|---|---|
| Amaranthus retroflexus | Zurückgekrümmter Fuschsschwanz | redroot pigweed |
| Arachis hypogaea | Erdnuß | peanuts (groundnuts) |
| Beta vulgaris | Zuckerrübe | sugarbeets |
| Centaura cyanus | Kornblume | cornflower |
| Chrysanthemum segetum | Saatwucherblume | corn marigold |
| Datura stramonium | Gemeiner Stechapfel | Jimsonweed |
| Echinochloa crus galli | Hühnerhirse | barnyardgrass |
| Glycine max | Sojabohnen | soybeans |
| Ipomoea spp. | Prunkwindearten | morningglory |
| Lolium multiflorum | Ital. Raygras | annual raygrass |
| Matricaria spp. | Kamillearten | chamomille |
| Nicandra physaloides | Giftbeere | apple-of-Peru |
| Sesbania exaltata | Turibaum | hemp sesbania (coffeeweed) |
| Sinapis alba | Weißer Gänsefuß | white mustard |

Fortsetzung

| Botanischer Name | Deutscher Name | Englischer Name |
|---|---|---|
| Sorghum bicolor | Mohrenhirse (Kulturhirse) | sorghum |
| Solanum nigrum | Schwarzer Nachtschatten | black nightshade |
| Stellaria media | Vogelsternmiere | chickweed |
| Triticum aestivum | Weizen | wheat |

Tabelle 2

Selektive Bekämpfung unerwünschter Pflanzen in verschiedenen Kulturen bei Vorauflaufanwendung im Gewächshaus

| Testpflanzen | Wirkstoff-Nr. 11 | Vergleichsmittel (bekannt) 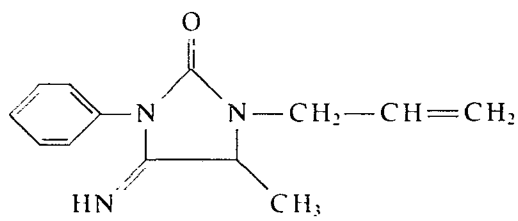 |
|---|---|---|
| | Aufwandmenge 2 kg Wirkstoff je ha | |
| Beta vulgaris | 0 | – |
| Glycine max | 0 | – |
| Sorghum bicolor | 0 | 20 |
| Amaranthus retroflexus | 100 | 90 |
| Chrysanthemum segetum | 95 | 100 |
| Echinochloa crus galli | 89 | 0 |
| Sesbania exaltata | 100 | 40 |
| Sinapis alba | 89 | 70 |
| Solanum nigrum | 100 | 90 |

0 = keine Schädigung
100 = Pflanzen abgestorben

Bemerkenswert ist die Schädigung der Nutzpflanze Sorghum und die fehlende oder schwache Wirkung auf die unerwünschten Pflanzen Echinochloa und Sesbania durch den bekannten Wirkstoff.

Tabelle 3

Selektive Bekämpfung unerwünschter Pflanzen bei Vorauflaufanwendung im Gewächshaus

Wirkstoff-Nr. 10

| Testpflanzen | Schädigung bei kg/ha | |
|---|---|---|
| | 1,0 | 2,0 |
| Arachis hypogaea | 0 | 10 |
| Sorghum bicolor | 0 | 5 |
| Amaranthus retroflexus | 90 | 100 |
| Chrysanthemum segetum | 85 | 100 |
| Datura stramonium | 100 | 100 |
| Sesbania exaltata | 98 | 98 |

0 = ohne Schädigung.
100 = kein Auflauf oder Pflanzen völlig abgestorben.

Tabelle 4

Selektive Bekämpfung von unerwünschten Pflanzen in Getreide und Sorghum bei Vorauflaufanwendung im Gewächshaus

Wirkstoff-Nr. 9

| Testpflanzen | Schädigung bei kg/ha |
|---|---|
| | 0,5 |
| Sorghum bicolor | 0 |
| Triticum aestivum | 5 |
| Amaranthus retraflexus | 79 |
| Matricaria spp. | 98 |
| Nicandra physaloides | 100 |
| Sesbania exaltata | 80 |
| Solanum nigrum | 80 |
| Stellaria media | 100 |

Tabelle 5

Selektive herbizide Wirkung bei Vorauflaufanwendung im Gewächshaus

| Testpflanzen | Wirkstoff-Nr. 1 | 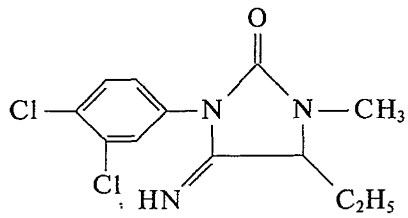 Vergleichssubstanz (bekannt) |
|---|---|---|
| | Schädigung bei 0,5 kg/ha | |
| Arachis hypogaea | 8 | 0 |
| Beta vulgaris | 0 | – |
| Amaranthus retroflexus | 97 | 40 |
| Chrysanthemum segetum | 85 | 0 |
| Datura stramonium | 100 | 0 |
| Solanum nigrum | 100 | 70 |

0 = keine Schädigung
100 = Pflanzen nicht aufgelaufen oder abgestorben

Tabelle 6

Herbizide Aktivität neuer Verbindungen bei Vor- und Nachauflaufanwendung im Gewächshaus

| Wirkstoff-Nr. | Testpflanzen und Schädigung bei 3,0 kg/ha | | | | |
|---|---|---|---|---|---|
| | Vorauflaufanwendung | | Nachauflaufanwendung | | |
| | Lolium multiflorum | Sinapis alba | Echinochloa crus galli | Ipomoea spp. | Centaurea cyanus |
| 3 | 95 | 100 | – | 100 | 100 |
| 4 | 95 | 100 | 100 | 100 | 100 |
| 5 | 70 | 100 | 100 | 100 | 100 |
| 6 | 80 | 100 | 95 | 90 | 100 |
| 7 | 90 | 100 | 80 | – | 100 |
| 8 | 90 | 100 | 80 | – | 100 |
| 12 | 100 | 100 | 90 | – | 100 |
| 13 | – | 80 | 60 | 100 | 90 |
| 14 | 95 | 100 | 90 | – | 100 |
| 15 | 100 | 100 | 95 | 100 | 100 |
| 16 | 100 | 100 | 100 | 100 | 100 |
| 17 | 60 | 90 | 70 | 95 | 100 |
| 19 | 100 | 100 | 90 | 90 | 100 |
| 20 | 90 | 100 | 95 | 90 | 90 |
| 22 | 60 | 100 | 90 | 100 | 95 |
| 23 | – | 95 | 100 | 90 | 98 |
| 24 | – | 100 | – | 100 | 100 |
| 25 | 95 | 100 | 100 | 70 | 70 |
| 26 | – | 70 | 70 | 80 | 100 |

0 = keine Schädigung.
100 = Pflanzen nicht aufgelaufen oder abgestorben.

### Beispiel A

Man vermischt 90 Gewichtsteile der Verbindung 1 mit 10 Gewichtsteilen N-Methyl-pyrrolidon und erhält eine Lösung, die zur Anwendung in Form kleinster Tropfen geeignet ist.

### Beispiel B

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Rizinusöl besteht. Durch

Ausgießen und feines Verteilen der Lösung in 100 000 Gewichtsteilen Wasser erhält man eine wäßrige Dispersion, die 0,02 Gew.-% des Wirkstoffs enthält.

Beispiel C

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Rizinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel D

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel E

20 Gewichtsteile des Wirkstoffs 2 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

Beispiel F

3 Gewichtsteile der Verbindung 3 werden mit 97 Gewichtsteilen feinteiligem Kaolin innig vermischt. Man erhält auf diese Weise ein Stäubemittel, das 3 Gew.-% des Wirkstoffs enthält.

Beispiel G

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel H

40 Gewichtsteile des Wirkstoffs 1 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

Beispiel K

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäureharnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, LI, DE, FR, GB, IT, NL, SE**

1. 5-Imino-imidazolidin-2-on der allgemeinen Formel

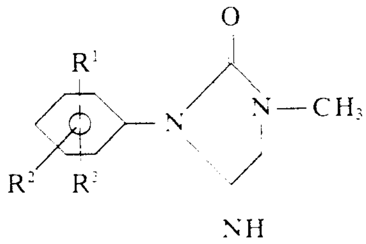

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

2. Herbizid, enthaltend ein 5-Imino-imidazolidin-2-on der allgemeinen Formel

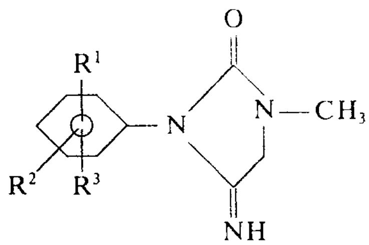

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atome, Cyan oder Nitro bedeuten.

3. Herbizid, enthaltend einen festen oder flüssigen Trägerstoff und ein 5-Imino-imidazolidin-2-on der allgemeinen Formel

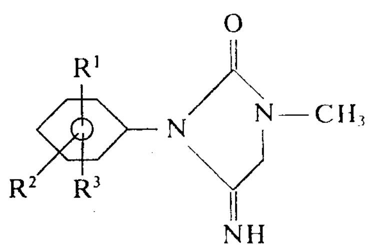

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

4. Verfahren zur Herstellung eines Herbizids, dadurch gekennzeichnet, daß man einen festen oder flüssigen Trägerstoff vermischt mit einem 5-Imino-imidazolidin-2-on der allgemeinen Formel

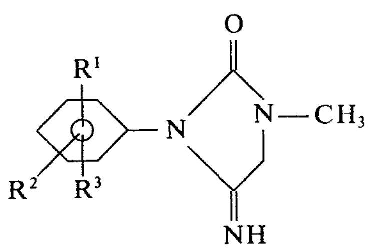

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

5. Verfahren zur Bekämpfung unerwünschten Pflanzenwuchses, dadurch gekennzeichnet, daß man die Pflanzen oder den Boden behandelt mit einem 5-Imino-imidazolidin-2-on der Formel

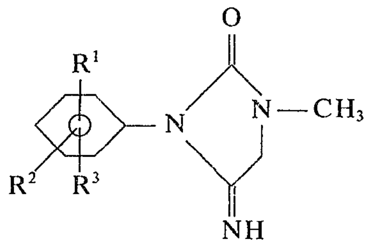

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

6. 5-Imino-imidazolidin-2-on, ausgewählt aus der Gruppe bestehend aus

1-(2-Fluorphenyl)-3-methyl-5-imino-imidazolidin-2-on,
1-(4-Isopropyl-phenyl)-3-methyl-5-imino-imidazolidin-2-on,
1-[3-(1,1,2,2-Tetrafluor-ethoxy)-phenyl]-3-methyl-5-imino-imidazolidin-2-on,
1-(3,4-Dichlorphenyl)-3-methyl-5-imino-imidazolidin-2-on,
1-(3,4-Difluorphenyl)-3-methyl-5-imino-imidazolidin-2-on.

7. Herbizid, enthaltend ein 5-Imino imidazolidin-2-on, ausgewählt aus der Gruppe bestehend aus

1-(2-Fluorphenyl)-3-methyl-5-iminoimidazolidin-2-on,
1-(4-Isopropyl-phenyl)-3-methyl-5-imino-imidazolidin-2-on,
1-[3-(1,1,2,2-Tetrafluor-ethoxy)-phenyl]-3-methyl-5-imino-imidazolidin-2-on,
1-(3,4-Dichlorphenyl)-3-methyl-5-imino-imidazolidin-2-on,
1-(3,4-Difluorphenyl)-3-methyl-5-imino-imidazolidin-2-on.

8. Herbizid, enthaltend einen festen oder flüssigen Trägerstoff und ein 5-Imino-imidazolidin-2-on, ausgewählt aus der Gruppe bestehend aus

1-(2-Fluorphenyl)-3-methyl-5-imino-imidazolidin-2-on,
1-(4-Isopropyl-phenyl)-3-methyl-5-imino-imidazolidin-2-on,
1,[3-(1,1,2,2-Tetrafluor-ethoxy)-phenyl]-3-methyl-5-imino-imidazolidin-2-on,
1-(3,4-Dichlorphenyl)-3-methyl-5-imino-imidazolidin-2-on,
1-(3,4-Difluorphenyl)-3-methyl-5-imino-imidazolidin-2-on.

**Patentansprüche für den Vertragsstaat: AT**

1. Herbizid, enthaltend ein 5-Imino-imidazolidin-2-on der allgemeinen Formel

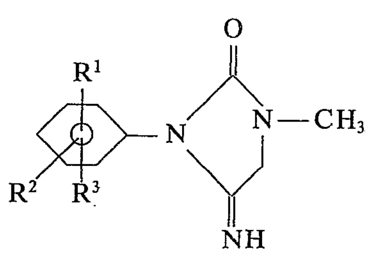

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atome, Cyan oder Nitro bedeuten.

2. Herbizid, enthaltend einen festen oder flüssigen Trägerstoff und ein 5-Imino-imidazolidin-2-on der allgemeinen Formel

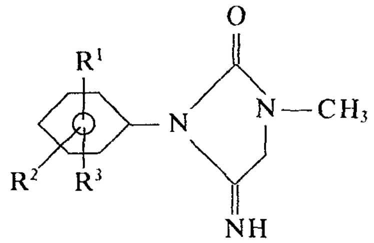

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

3. Verfahren zur Herstellung eines Herbizids, dadurch gekennzeichnet, daß man einen festen oder flüssigen Trägerstoff vermischt mit einem 5-Imino-imidazolidin-2-on der allgemeinen Formel

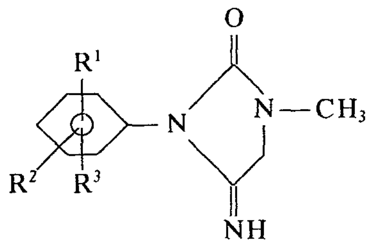

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

4. Verfahren zur Bekämpfung unerwünschten Pflanzenwuchses, dadurch gekennzeichnet, daß man die Pflanzen oder den Boden behandelt mit einem 5-Imino-imidazolidin-2-on der Formel

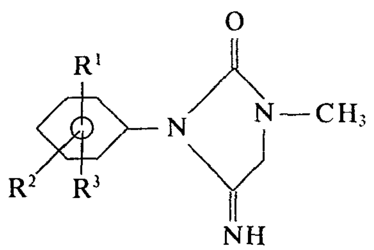

in welcher $R^1$, $R^2$ und $R^3$ gleich oder verschieden sind und unabhängig voneinander Wasserstoff, Halogen, eine gegebenenfalls halogensubstituierte Alkyl-, Alkoxy- oder Alkyl-thio-Gruppe mit jeweils 1 bis 4 C-Atomen, Cyan oder Nitro bedeuten.

5. Herbizid, enthaltend ein 5-Imino-imidazolidin-2-on, ausgewählt aus der Gruppe bestehend aus

1-(2-Fluorphenyl)-3-methyl-5-iminoimidazolidin-2-on,
1-(4-Isopropyl-phenyl)-3-methyl-5-imino-imidazolidin-2-on,
1-[3-(1,1,2,2-Tetrafluor-ethoxy)-phenyl]-3-methyl-5-imino-imidazolidin-2-on,
1-(3,4-Dichlorphenyl)-3-methyl-5-imino-imidazolidin-2-on,
1-(3,4-Difluorphenyl)-3-methyl-5-imino-imidazolidin-2-on.

6. Herbizid, enthaltend einen festen oder flüssigen Trägerstoff und ein 5-Imino-imidazolidin-2-on, ausgewählt aus der Gruppe bestehend aus

1-(2-Fluorphenyl)-3-methyl-5-imino-imidazolidin-2-on,
1-(4-Isopropyl-phenyl)-3-methyl-5-imino-imidazolidin-2-on,
1-[3-(1,1,2,2-Tetrafluor-ethoxy)-phenyl]-3-methyl-5-imino-imidazolidin-2-on,
1-(3,4-Dichlorphenyl)-3-methyl-5-imino-imidazolidin-2-on,
1-(3,4-Difluorphenyl)-3-methyl-5-imino-imidazolidin-2-on.

**Claims for the contracting states BE, CH, LI, DE, FR, GB, IT, NL, SE**

1. A 5-imino-imidazolin-2-one of the general formula

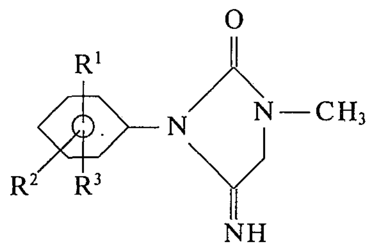

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

2. A herbicide containing a 5-imino-imidazolidin-2-one of the general formula

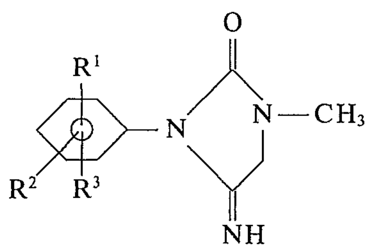

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

3. A herbicide containing a solid or liquid carrier and a 5-imino-imidazolidin-2-one of the general formula

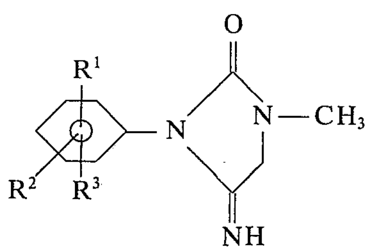

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

4. A process for the production of a herbicide, characterized in that a solid or liquid carrier is mixed with a 5-imino-imidazolidin-2-one of the general formula

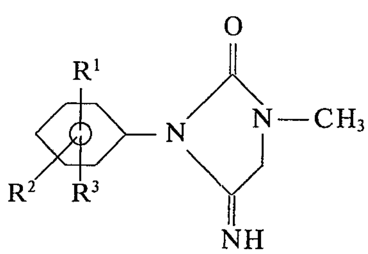

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

5. A process for combating unwanted plant growth, characterized in that the plants or the soil are treated with a 5-imino-imidazolidin-2-one of the formula

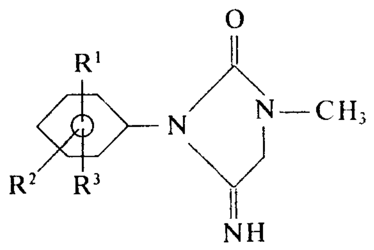

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

6. A 5-imino-imidazolidin-2-one selected from the group consisting of

1-(2-fluorophenyl)-3-methyl-5-imino-imidazolidin-2-one,
1-(4-isopropylphenyl)-3-methyl-5-imino-imidazolidin-2-one,
1-[3-(1,1,2,2-tetrafluoroethoxy)-phenyl]-3-methyl-5-imino-imidazolidin-2-one,
1-(3,4-dichlorophenyl)-3-methyl-5-imino-imidazolidin-2-one and
1-(3,4-difluorophenyl)-3-methyl-5-imino-imidazolidin-2-one.

7. A herbicide containing a 5-imino-imidazolidin-2-one selected from the group consisting of

1-(2-fluorophenyl)-3-methyl-5-imino-imidazolidin-2-one,
1-(4-isopropylphenyl-3-methyl-5-imino-imidazolidin-2-one,
1-[3-(1,1,2,2-tetrafluoroethoxy)-phenyl]-3-methyl-5-imino-imidazolidin-2-one,
1-(3,4-dichlorophenyl)-3-methyl-5-imino-imidazolidin-2-one and
1-(3,4-difluorophenyl)-3-methyl-5-imino-imidazolidin-2-one.

8. A herbicide containing a solid or liquid carrier and a 5-imino-imidazolidin-2-one selected from the group consisting of

1-(2-fluorophenyl)-3-methyl-5-imino-imidazolidin-2-one,
1-(4-isopropylphenyl)-3-methyl-5-imino-imidazolidin-2-one,
1-[3-(1,1,2,2-tetrafluoroethoxy)-phenyl]-3-methyl-5-imino-imidazolidin-2-one,
1-(3,4-dichlorophenyl)-3-methyl-5-imino-imidazolidin-2-one and
1-(3,4-difluorophenyl)-3-methyl-5-imino-imidazolidin-2-one.

**Claims for Austria**

1. A herbicide containing a 5-imino-imidazolidin-2-one of the general formula

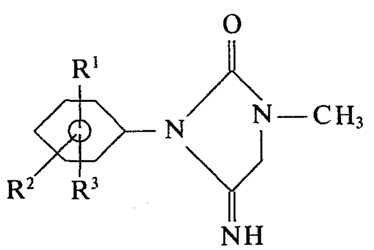

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

2. A herbicide containing a solid or liquid carrier and a 5-imino-imidazolidin-2-one of the general formula

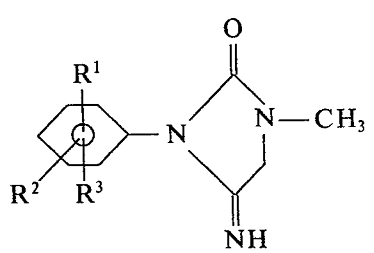

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

3. A process for the production of a herbicide, characterized in that a solid or liquid carrier is mixed with a 5-imino-imidazolidin-2-one of the general formula

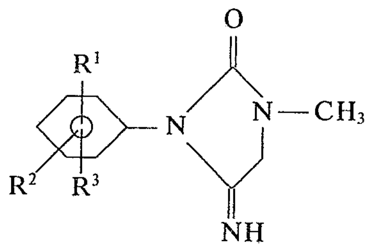

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

4. A process for combating unwanted plant growth, characterized in that the plants or the soil are treated with a 5-imino-imidazolidin-2-one of the formula

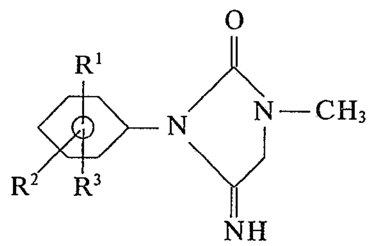

where $R^1$, $R^2$ and $R^3$ are identical or different and, independently of one another, denote hydrogen, halogen, unsubstituted or halogen-substituted alkyl, alkoxy or alkylthio, each of from 1 to 4 carbon atoms, cyano or nitro.

5. A herbicide containing a 5-imino-imidazolidin-2-one selected from the group consisting of

1-(2-fluorophenyl)-3-methyl-5-imino-imidazolidin-2-one,
1-(4-isopropylphenyl)-3-methyl-5-imino-imidazolidin-2-one,
1-[3-(1,1,2,2-tetrafluoroethoxy)-phenyl]-3-methyl-5-imino-imidazolidin-2-one,
1-(3,4-dichlorophenyl)-3-methyl-5-imino-imidazolidin-2-one and
1-(3,4-difluorophenyl)-3-methyl-5-imino-imidazolidin-2-one.

6. A herbicide containing a solid or liquid carrier and a 5-imino-imidazolidin-2-one selected from the group consisting of

1-(2-fluorophenyl)-3-methyl-5-imino-imidazolidin-2-one,
1-(4-isopropylphenyl)-3-methyl-5-imino-imidazolidin-2-one,
1-[3-(1,1,2,2-tetrafluoroethoxy)-phenyl]-3-methyl-5-imino-imidazolidin-2-one,
1-(3,4-dichlorophenyl)-3-methyl-5-imino-imidazolidin-2-one and
1-(3,4-difluorophenyl)-3-methyl-5-imino-imidazolidin-2-one.

**Revendications pour les états contractants BE, CH, LI, DE, FR, GB, IT, NL, SE**

1. 5-imino-imidazolidin-2-one de formule générale

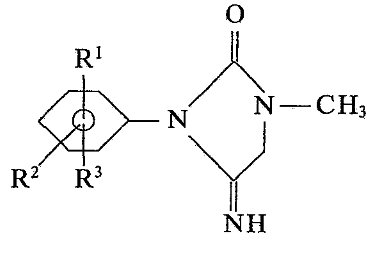

dans laquelle $R^1$, $R^2$ et $R^3$ sont identiques ou différents et représentent, indépendamment l'un de

l'autre, hydrogène, halogène, cyano, nitro ou un groupe alkyl-, alcoxy- ou alkylthio à 1 à 4 atomes C, éventuellement substitué par un halogène.

2. Herbicide, contenant une 5-imino-imidazolidin-2-one de formule générale

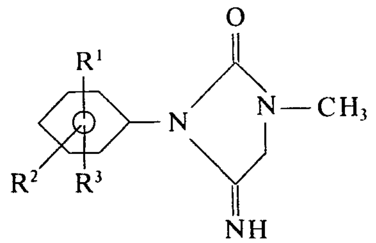

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

3. Herbicide contenant un support solide ou liquide et une 5-imino-imidazolidin-2-one de formule générale

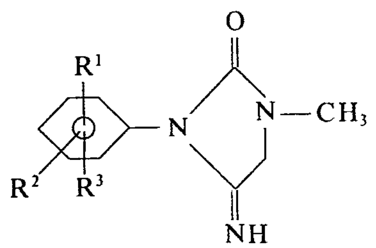

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

4. Procédé de préparation d'un herbicide, caractérisé par le fait qu'on mélange un support solide ou liquide avec une 5-imino-imidazolidin-2-one de formule générale

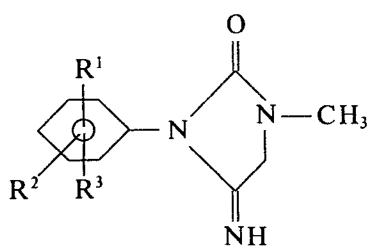

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

5. Procédé pour lutter contre la croissance indésirable de plantes, caractérisé par le fait qu'on traite les plantes ou le sol avec une 5-imino-imidazolidin-2-one de formule générale

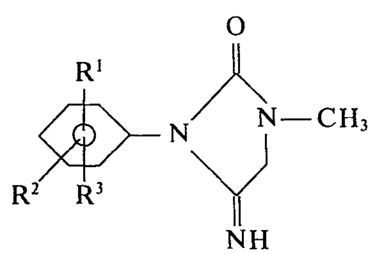

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

6. 5-imino-imidazolidin-2-one, choisie dans le groupe constitué de

1-(2-fluorophényl)-3-méthyl-5-imino-imidazolidin-2-one,
1-(4-isopropyl-phényl)-3-méthyl-5-imino-imidazolidin-2-one,

1-[3-(1,1,2,2-tétrafluor-éthoxy)-phényl]-3-méthyl-5-imino-imidazolidin-2-one,
1-(3,4-dichlorphényl)-3-méthyl-5-imino-imidazolidin-2-one,
1-(3,4-difluorophényl)-3-méthyl-5-imino-imidazolidin-2-one.

7. Herbicide contenant une 5-imino-imidazolidin-2-one choisie dans le groupe constitué de

1-(2-fluorophényl)-3-méthyl-5-imino-imidazolidin-2-one;
1-(4-isopropyl-phényl)-3-méthyl-5-imino-imidazolidin-2-one,
1-[3-(1,1,2,2-tétrafluor-éthoxy)-phényl]-3-méthyl-5-imino-imidazolidin-2-one,
1-(3,4-dichlorophényl)-3-méthyl-5-imino-imidazolidin-2-one,
1-(3,4-difluorophényl)-3-méthyl-5-imino-imidazolidin-2-one.

8. Herbicide contenant un support solide ou liquide et une 5-imino-imidazolidin-2-one, choisie dans le groupe constitué de

1-(2-fluorophényl)-3-méthyl-5-imino-imidazolidin-2-one,
1-(4-isopropyl-phényl)-3-méthyl-5-imino-imidazolidin-2-one,
1-[3-(1,1,2,2-tétrafluor-éthoxy)-phényl]-3-méthyl-5-imino-imidazolidin-2-one,
1-(3,4-dichlorophényl)-3-méthyl-5-imino-imidazolidin-2-one,
1-(3,4-difluorophényl)-3-méthyl-5-imino-imidazolidin-2-one.

**Revendications pour l'Autriche**

1. Herbicide contenant une 5-imino-imidazolidin-2-one de formule générale

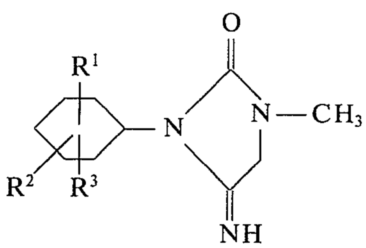

dans laquelle $R^1$, $R^2$ et $R^3$ sont identiques ou différents et représentent indépendamment l'un de l'autre, hydrogène, halogène, cyano, nitro ou un groupe alkyl-, alcoxy- ou alkylthio à 1 à 4 atomes C, éventuellement substitué par un halogène.

2. Herbicide contenant un support solide ou liquide et une 5-imino-imidazolidin-2-one de formule générale

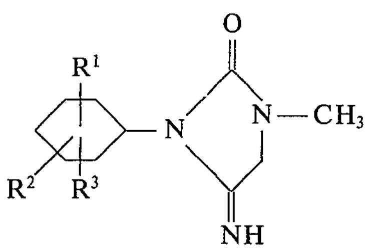

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

3. Procédé de préparation d'un herbicide, caractérisé par le fait qu'on mélange un support solide ou liquide avec une 5-imino-imidazolidin-2-one de formule générale

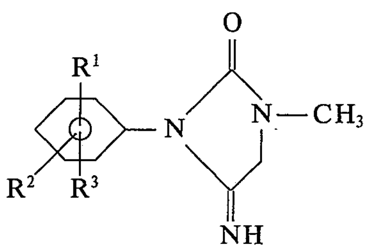

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

4. Procédé pour lutter contre la croissance indésirable de plantes, caractérisé par le fait qu'on traite les plantes ou le sol avec une 5-imino-imidazolidin-2-one de formule générale

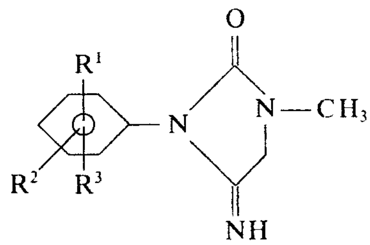

dans laquelle $R^1$, $R^2$ et $R^3$ sont identiques ou différents et représentent, indépendamment l'un de l'autre, hydrogène, halogène, cyano, nitro ou un groupe alkyl-, alcoxy- ou alkylthio à 1 à 4 atomes C, éventuellement substitué par un halogène.

5. Herbicide contenant une 5-imino-imidazolidin-2-one, choisie dans le groupe constitué de

1-(2-fluorophényl)-3-méthyl-5-imino-imidazolidin-2-one;
1-(4-isopropyl)-phényl)-3-méthyl-5-imino-imidazolidin-2-one,
1-[3-(1,1,2,2-tétrafluor-éthoxy)-phényl]-3-méthyl-5-imino-imidazolidin-2-one,
1-(3,4-dichlorophényl)-3-méthyl-5-imino-imidazolidin-2-one,
1-(3,4-difluorophényl)-3-méthyl-5-imino-imidazolidin-2-one.

6. Herbicide contenant un support solide ou liquide et une 5-imino-imidazolidin-2-one, choisie dans le groupe constitué de

1-(2-fluorophényl)-3-méthyl-5-imino-imidazolidin-2-one,
1-(4-isopropylphényl)-3-méthyl-5-imino-imidazolidin-2-one,
1-[3-(1,1,2,2-tétrafluor-éthoxy)-phényl]-3-méthyl-5-imino-imidazolidin-2-one,
1-(3,4-dichlorophényl)-3-méthyl-5-imino-imidazolidin-2-one,
1-(3,4-difluorophényl)-3-méthyl-5-imino-imidazolidin-2-one.